# EUROPÄISCHE PATENTSCHRIFT

(11) **EP 0 774 120 B1**
(45) Veröffentlichungstag und Bekanntmachung des Hinweises auf die Patenterteilung: **26.08.2009**
(21) Anmeldenummer: 95927714.6
(22) Anmeldetag: 24.07.1995
(51) Int. Cl.: G01N 33/533, G01N 33/58, C07F 15/00

(54) **METALLCHELAT-MARKIERTE PEPTIDE**
PEPTIDES MARKED WITH METAL CHELATES
PEPTIDES MARQUES AVEC DES CHELATES METALLIQUES

(30) Priorität: 25.07.1994 DE 4426276; 31.08.1994 DE 4430998
(43) Veröffentlichungstag der Anmeldung: 21.05.1997
(73) Patentinhaber: Roche Diagnostics GmbH, 68305 Mannheim (DE)
(72) Erfinder: SEIDEL, Christoph, D-82362 Weilheim (DE); WIENHUES, Ursula-Henrike, D-82152 Krailling (DE); HÖSS, Eva, D-82319 Starnberg (DE)
(74) Vertreter: Weiss, Wolfgang
(86) Internationale Anmeldenummer: PCT/EP1995/002916
(87) Internationale Veröffentlichungsnummer: WO 1996/003651

(56) Entgegenhaltungen:
- EP-A- 0 178 450
- EP-A- 0 280 211
- EP-A- 0 438 332
- EP-A- 0 580 979
- WO-A-86/02734
- WO-A-92/22571
- WO-A-93/06488
- WO-A-93/18054
- US-A- 4 849 505
- ARYA R. AND GARIÉPY J.: "RAPID SYNTHESIS AND INTRODUCTION OF A PROTECTED EDTA-LIKE GROUP DURING THE SOLID-PHASE ASSEMBLY OF PEPTIDES" BIOCONJUGATE CHEMISTRY, Bd. 2, Nr. 5, 1991, Seiten 323-326, USA
- JAVAHERIAN K; ET AL: "PRINCIPAL NEUTRALIZING DOMAIN OF THE HUMAN IMMUNODEFICIENCY VIRUS TYPE 1 ENVELOPE PROTEIN" PROCEEDINGS OF THE NATIONAL ACADEMY OF SCIENCES OF USA, NATIONAL ACADEMY OF SCIENCE, Bd. 86, Nr. 17, 1. September 1989 (1989-09-01), Seiten 6768-6772, WASHINGTON, DC, US
- YAIR DEVASH; ET AL: "C-TERMINAL FRAGMENTS OF GP120 AND SYNTHETIC PEPTIDES FROM FIVE HTLV-III STRAINS: PREVALENCE OF ANTIBODIES TO THE HTLV-III-MN ISOLATE IN INFECTED INDIVIDUALS" AIDS RESEARCH AND HUMAN RETROVIRUSES, Bd. 6, Nr. 3, 1. Januar 1990 (1990-01-01), Seiten 307-316, NEW YORK, NY, US
- MCCAFFERTY DEWEY G; BISHOP BARNEY M; WALL CRAIG G; HUGHES SOLON G; MECKLENBERG SANDRA L; MEYER THOMAS J; ERICKSON BRUCE W: "Synthesis of redox derivatives of lysine and their use in solid-phase synthesis of a light-harvesting peptide" TETRAHEDRON, Bd. 51, Nr. 4, 23. Januar 1995 (1995-01-23), Seiten 1093-1106, AMSTERDAM, NL
- BLACKBURN G F; SHAH H P; KENTEN J H; LELAND J; KAMIN R A; LINK J; PETERMAN J; POWELL M J; SHAH A; TALLEY D B; ET AL.: "Electrochemiluminescence detection for development of immunoassays and DNA probe assays for clinical diagnostics." CLINICAL CHEMISTRY, Bd. 37, Nr. 9, 1. September 1991 (1991-09-01), Seiten 1534-1539, WASHINGTON, DC, US
- PEEK B M; ROSS G T; EDWARDS S W; MEYER G J; MEYER T J; ERICKSON B W: "SYNTHESIS OF REDOX DERIVATIVES OF LYSINE AND RELATED PEPTIDES CONTAINING PHENOTHIAZINE OR TRIS-2 2'-BIPYRIDINE-RUTHENIUM-II" INTERNATIONAL JOURNAL OF PEPTIDE AND PROTEIN RESEARCH, Bd. 38, Nr. 2, 1991, Seiten 114-123,
- WATTS T H; GARIÉPY J; SCHOOLNIK G K; MCCONNELL H M: "T-cell activation by peptide antigen: effect of peptide sequence and method of antigen presentation." PROCEEDINGS OF THE NATIONAL ACADEMY OF SCIENCES OF THE UNITED STATES OF AMERICA, Bd. 82, Nr. 16, 1. August 1985 (1985-08-01), Seiten 5480-5484, UNITED STATES
- STOEVA; STANKA; GRUEBLER; GERALD; ECHNER; HARTMUT; ROENSPECK; WOLFGANG; VOELTER; WOLFGANG: "HIV-transmembrane glycoprotein GP41 ENV fragments with high antigenicity andtheir application to determine HIV-positive sera" PURE & APPLIED CHEMISTRY, Bd. 66, Nr. 1, 1. Januar 1994 (1994-01-01), Seiten 101-104, OXFORD, GB

## Beschreibung

Die vorliegende Erfindung betrifft ein Verfahren zur Herstellung von Metallchelat-markierten Peptiden, durch dieses Verfahren erhältliche Metallchelat-markierte Peptide sowie die Verwendung dieser Peptide in einem immunologischen Nachweisverfahren.

Der Nachweis von Immunglobulinen in Körperflüssigkeiten, insbesondere in Humanseren, wird zur Diagnostik von Infektionen mit Mikroorganismen, insbesondere Viren, wie etwa HIV, Hepatitis-Viren, etc. verwendet. Das Vorhandensein von spezifischen Immunglobulinen in der untersuchten Probe wird üblicherweise durch Reaktion mit einem oder mehreren Antigenen, die mit den spezifischen Immunglobulinen reagieren, nachgewiesen. Verfahren zur Bestimmung von spezifischen Immunglobulinen in der Probeflüssigkeit müssen sensitiv, zuverlässig, einfach und schnell sein.

In den letzten Jahren wurden zunehmend Nachweissysteme auf Basis nicht-radioaktiver Markierungsgruppen entwickelt, bei denen das Vorhandensein eines Analyten, z.B. eines spezifischen Antikörpers, in der untersuchten Probe mit Hilfe optischer (z.B. Lumineszenz oder Fluoreszenz), NMR-aktiver oder Metallpräzipitierender Detektionssysteme bestimmt werden konnte.

EP-A-0 307 149 offenbart einen Immuntest für einen Antikörper, bei dem zwei rekombinante Polypeptide als Antigene verwendet werden, von denen eines an einer festen Phase immobilisiert ist, und das andere eine Markierungsgruppe trägt, wobei beide rekombinanten Antigene in unterschiedlichen Organismen exprimiert werden, um die Spezifität des Nachweises zu erhöhen.

EP-A-0 366 673 offenbart ein Verfahren zum Nachweis von Antikörpern in einer Probe, bei dem ein Antikörper durch Reaktion mit einem gereinigten, markierten Antigen und dem gleichen gereinigten Antigen in einer Festphasen-gebundenen Form nachgewiesen wird. Als Antigen wird beispielsweise humanes IgG offenbart.

EP-A-0 386 713 beschreibt ein Verfahren zum Nachweis von Antikörpern gegen HIV unter Verwendung von zwei festen Trägern, wobei an beide festen Träger verschiedene HIV-Antigene immobilisiert werden, die jeweils mit einem Aliquot einer Probe und einem markierten HIV-Antigen in Kontakt gebracht werden, wobei das Vorhandensein von Antikörpern durch eine positive Reaktion in mindestens einem der Tests nachgewiesen wird. Als HIV-Antigene werden rekombinant hergestellte Polypeptide offenbart.

EP-A-0 507 586 beschreibt ein Verfahren zur Durchführung eines immunologischen Tests für ein spezifisches Immunglobulin, bei dem eine Probe mit zwei zur Bindung des Immunglobulins fähigen Antigenen in Kontakt gebracht wird, wobei das erste Antigen eine zur Bindung an einen festen Träger geeignete Gruppe trägt, und das zweite Antigen eine Markierungsgruppe trägt. Die Markierungsgruppe kann eine direkte Markierungsgruppe sein, z.B. ein Enzym, ein Chromogen, ein Metallteilchen, oder auch eine indirekte Markierungsgruppe, d.h. die am Antigen angebrachte Markierungsgruppe kann mit einem Rezeptor für die Markierungsgruppe, der wiederum eine signalerzeugende Gruppe trägt, reagieren. Als Beispiel für eine solche indirekte Markierungsgruppe wird ein Fluoresceinderivat genannt, dessen Rezeptor ein Antikörper ist, der wiederum mit einem Enzym gekoppelt ist. Als Antigene werden Polypeptide, wie etwa das Hepatitis B-Oberflächenantigen offenbart. In dieses Antigen werden durch Derivatisierung SH-Gruppen eingeführt, mit denen das Fluorescein gekoppelt wird.

EP-A-0 507 587 offenbart ein spezifisch zum Nachweis von IgM Antikörpern geeignetes Verfahren, bei dem die Probe mit einem markierten Antigen, das gegen den nachzuweisenden Antikörper gerichtet ist, und einem zweiten Antikörper, der ebenfalls gegen den nachzuweisende Antikörper gerichtet und an eine Festphase bindefähig ist, inkubiert wird.

EP-A-0 199 804 und EP-A-0 580 979 offenbaren ein immunologisches Nachweisverfahren unter Verwendung von Antigenen, die mit lumineszierenden Metallchelatgruppen, insbesondere mit Ruthenium- und Osmiumchelatgruppen markiert sind. Als Antigene werden Immunglobuline verwendet; die durch Reaktion mit aktivierten Metallkomplexen statistisch markiert werden.

EP-A-0 178 450 offenbart Metallchelate, insbesondere Rutheniumkomplexe, an die ein immunologisch aktives Material, beispielsweise Antikörper, gekoppelt werden kann. Die Kopplung erfolgt durch statistische Realtion des immunologisch reaktiven Materials mit dem Metallchelat.

EP-A-0 255 534 offenbart einen Lumineszenz-Immunoassay unter Verwendung eines Metallchelat-gekoppelten Antigene oder Antikörpers. Die Kopplung erfolgt beispielsweise durch statistische Reaktion eines Metallchelat-Aktivesterderivats mit einem Antikörper.

WO 90/05301 offenbart ein Verfahren zum Nachweis' und zur quantitativen Bestimmung von Analyten durch Elektrochemilumineszenz unter Verwendung von lumineszierenden Metallchelaten, die an (i) einen zugesetzten Analyten, (ii) einen Bindepartner des Analyten oder (iii) eine reaktive Komponente, die mit (i) oder (ii) binden kann, gekoppelt sind. Die Lumineszenzmessung erfolgt nach Bindung der Metallchelate an aktivierte und gegebenenfalls magnetische Mikropartikel.
Blocksburn et al. (Clin. Chem. 37/ 9, 1534-1539, 1991) beschreibt Metallchelat-markierte Antikörper und deren Verwendung in Immuntests.
Bei den aus dem Stand der Technik bekannten immunologischen Nachweisverfahren für Antikörper werden üblicherweise Polypeptid-Antigene verwendet, die meist durch rekombinante DNA-Methoden erzeugt wurden. Beim Einsatz derartiger Polypeptid-Antigene können jedoch Probleme auftreten. So können rekombinante Polypetide oft nur in Form von Fusionspolypeptiden erzeugt werden, bei denen der Fusionsanteil zu falsch positiven Resultaten im Test führen kann. Weiterhin zeigen durch rekombinante Expression erzeugte Polypeptide of eine nur geringe Stabilität in der Probelösung und neigen zu Aggregation. Ein weiterer Nachteil ist, daß oft keine selektive und reproduzierbare Einführung von Markierungsgruppen in solche Polypeptide möglich ist.

Überdies ist die Herstellung rekombinanter Polypeptidantigene mit hohen Kosten verbunden und es können größere Schwankungen in der immunologischen Reaktivität bei verschiedenen Chargen rekombinanter Polypeptide auftreten.

Das der vorliegenden Erfindung zugrundeliegende Problem war somit die Bereitstellung eines Verfahrens, bei dem auf einfache und effiziente Weise Antigene für immunologische Tests erzeugt werden können, wobei die Nachteile der aus dem Stand der Technik bekannten Antigene mindestens teilweise beseitigt werden. Weiterhin soll das Verfahren eine selektive und reproduzierbare Einführung von Markierungsgruppen in die Antigene ermöglichen.

Dieses Problem wird gelöst durch ein Verfahren zur Herstellung von Metallchelat-markierten Peptiden, welches dadurch gekennzeichnet ist,
daß man ein Peptid mit der gewünschten Aminosäuresequenz an einer Festphase synthetisiert, das einen immunologisch reaktiven Epitopbereich und einen Spacerbereich umfasst,
wobei man (a) nach der Synthese ein aktiviertes lumineszierendes Metallchelat an die N-terminale primäre Aminogruppe des Peptids koppelt, oder/und
(b) während der Synthese an mindestens einer Position des Peptids ein Aminosäurederivat einführt, das kovalent mit einer lumineszierenden Metallchelat-Markierungsgruppe gekoppelt ist,
wobei mindestens eine Metallchelat-Markierung an den Spacerbereich gekoppelt wird und wobei die Liganden in den Metallchelaten ausgewählt werden aus aromatischen heterocyclischen Polydentatliganden.

Die durch das erfindungsgemäße Verfahren hergestellten Peptide haben vorzugsweise eine Länge von maximal 50 Aminosäuren, besonders bevorzugt von maximal 30 Aminosäuren und eignen sich hervorragend für immunologische Nachweisverfahren, insbesondere zur Bestimmung von spezifischen Immunglobulinen. Überraschenderweise wurde festgestellt, daß die durch das erfindungsgemäße Verfahren hergestellten Peptide trotz der Anwesenheit von sperrigen Metallchelat-Markierungsgruppen eine hohe Affinität und Spezifität für die nachzuweisenden Immunglobuline besitzen.

Das erfindungsgemäße Verfahren ermöglicht die selektive Einführung von Metallchelat-Markierungsgruppen, sowohl bezüglich ihrer Lokalisierung als auch ihrer Anzahl. Bei der erfindungsgemäßen Peptidsynthese besteht nämlich die Möglichkeit, durch selektiven Einbau von Metallchelat-markierten Aminosäurederivaten diejenigen Positionen des Peptids gezielt auszuwählen, an die eine Markierung eingeführt wird. Auf diese Weise wird eine bessere Reproduzierbarkeit und Sensitivität von immunologischen Tests erreicht, in denen die erfindungsgemäß hergestellten Peptide verwendet werden.

Ein weiterer Vorteil des erfindungsgemäßen Verfahrens ist, daß durch die Verwendung von Peptid-Antigenen alle Antikörperklassen, wie etwa IgG, IgM, IgE und IgA, erkannt werden. Auch die Störanfälligkeit des Tests ist durch Verwendung definierter kleiner und stabiler Antigene, die nicht zur Aggregation neigen, geringer.

Die Metallchelate, die durch das erfindungsgemäße Verfahren an das Peptid gekoppelt werden, sind lumineszierende Metallchelate, d.h. Metallchelate, die eine nachweisbare Lumineszenzreaktion erzeugen. Der Nachweis dieser Lumineszenzreaktion kann beispielsweise durch Fluoreszenz- oder durch Elektrochemilumineszenzmessung erfolgen. Das Metall dieser Metallchelate ist beispielsweise ein Übergangsmetall oder ein Seltenerdenmetall. Vorzugsweise ist das Metall Ruthenium, Osmium, Rhenium, Iridium, Rhodium, Platin, Indium, Palladium, Molybdän, Techneticum, Kupfer, Chrom oder Wolfram. Besonders bevorzugt sind Ruthenium, Iridium, Rhenium und Osmium. Am meisten bevorzugt ist Ruthenium.

Die Liganden, die zusammen mit dem Metall das Metallchelat bilden, sind aromatische heterocyclische Polydentat-Liganden, d.h. Liganden mit mehreren Koordinationsstellen. Bevorzugte aromatische heterocyclische Liganden sind N-haltige Polyheterocyclen wie etwa z.B. Bipyridyl, Bipyrazyl, Terpyridyl und Phenanthrolyl. Diese Liganden können beispielsweise Substituenten wie etwa Alkyl, substituiertes Alkyl, Aryl, substituiertes Aryl, Aralkyl, Carboxylat, Carboxyaldehyd, Carboxamid, Cyano, Amino, Hydroxy, Imino, Hydroxycarbonyl, Aminocarbonyl, Amidin, Guanidinium, Ureid, schwefelhaltige Gruppen, phosphorhaltige Gruppen und die Carboxylatester von N-Hydroxysuccinimid umfassen. Das Chelat kann auch einen oder mehrere Monodentat-Liganden enthalten. Beispiele von Monodentat-Ligänden umfassen kohlenmonoxid, Cyanide, Isocyanide, Halogenide und aliphatische, aromatische und heterocyclische Phosphine, Amine, Stilbene und Arsine.

Besonders bevorzugt wird das lumineszierende Metallchelat ausgewählt aus Metallchelaten mit Bipyridyl- oder Phenanthrolyl-Liganden. Beispiele geeigneter Metallchelate und ihre Herstellung sind in EP-A-0 178 450, EP-A-0 255 534, EP-A-0 580 979 und WO 90/05301 beschrieben. Am meisten bevorzugte Metallchelate sind Ruthenium-(bipyridyl)₃-chelate. Diese Chelate sind in Form von Aktivesterderivaten kommerziell erhältlich, z.B. von Igen Inc. (Rockville, MD, USA).

Gemäß Variante (a) des erfindungsgemäßen Verfahrens erfolgt die Einführung der Metallchelat-Markierung in das Peptid nach Synthese der gewünschten Aminosäuresequenz durch selektive Reaktion der N-terminalen primären Aminogruppe des Peptids mit einem aktivierten Metallchelat, z.B. einem Metallchelat-Aktivesterderivat. Vorzugsweise findet die Kopplung des aktivierten Metallchelats vor der Abspaltung des Peptids von der Festphase und vor der Abspaltung von Schutzgruppen an rekativen Seitenketten der zur Peptidsynthese verwendeten Aminosäurederivate statt.

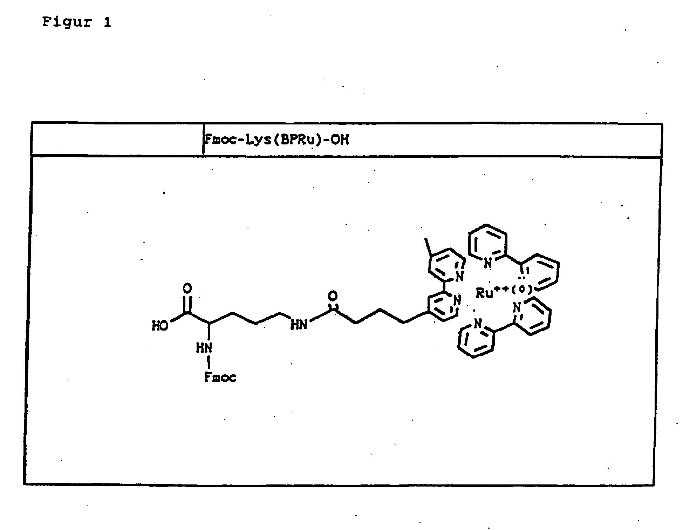

Bei Variante (b) des erfindungsgemäßen Verfahrens wird während der Festphasensynthese ein Aminosäurederivat eingeführt, das kovalent mit einer lumineszierenden Metallchelat-Markierungsgruppe gekoppelt ist. Vorzugsweise ist die Metallchelat-Markierungsgruppe an eine Aminogruppe, insbesondere an eine primäre Aminogruppe des Aminosäurederivats gekoppelt. Wenn die Markierungsgruppe während der Synthese am Aminoterminus der Peptidsequenz eingeführt werden soll, kann das Metallchelat an eine freie Aminogruppe der N-terminalen Aminosäure gekoppelt sein. Wenn die Markierungsgruppe innerhalb der Sequenz eingeführt werden soll, ist das Metallchelat vorzugsweise an die primäre Aminoseitengruppe einer Aminosäure wie etwa Lysin oder Ornithin gekoppelt. Die Herstellung von Aminosäure-Metallchelat-Derivaten kann beispielsweise durch Kopplung eines aktivierten Metallchelats, z.B. eines Metallchelat-Aktivesterderivats an eine freie primäre Aminogruppe eines gegebenenfalls partiell geschützten Aminosäurederivats erfolgen. In Fig. 1 ist ein bevorzugtes Metallchelat-gekoppeltes Lysinderivat gezeigt.

Der Begriff "Aktivester" im Sinne der vorliegenden Erfindung umfaßt aktivierte Estergruppen, die mit freien Aminogruppen von Peptiden unter solchen Bedingungen reagieren können, daß keine störenden Nebenreaktionen mit anderen reaktiven Gruppen des Peptids auftreten können. Vorzugsweise wird als Aktivesterderivat ein N-Hydroxysuccinimidester verwendet. Neben den N-Hydroxysuccinimidestern können auch analoge p-Nitrophenyl-, Pentafluorphenyl-, Imidazolyl- oder N-Hydroxybenzotriazolylester verwendet werden.

Bei dem erfindungsgemäßen Verfahren wird das Peptid mit der gewünschten Aminosäuresequenz an einer Festphase, vorzugsweise mit einem kommerziellen Peptid-Synthesegerät (z.B. die Geräte A 431 oder A 433 von Applied Biosystems) hergestellt. Die Synthese erfolgt nach bekannten Methoden, vorzugsweise ausgehend vom Carboxylterminus des Peptids unter Verwendung von Aminosäurederivaten. Vorzugsweise werden Aminosäurederivate eingesetzt, deren für die Kupplung benötigte Amino-Endgruppe mit einem Fluorenylmethyloxycarbonyl (Fmoc)-Rest derivatisiert ist. Reaktive Seitengruppen der eingesetzten Aminosäuren enthalten Schutzgruppen, die nach Beendigung der Peptidsynthese ohne weiteres abspaltbar sind. Bevorzugte Beispiele hierfür sind Schutzgruppen, wie etwa Triphenylmethyl (Trt), t-Butylether (tBu), t-Butylester (OtBu), tert.-Butoxycarbonyl (Boc) oder 2,2,5,7,8-Pentamethylchroman-6-sulfonyl (Pmc).

Die Aminoseitenketten von Lysinresten oder anderen Aminosäurederivaten mit primären Aminoseitengruppen, die sich an Positionen des Peptids befinden, an denen eine Markierung eingeführt werden soll, sind gemäß Variante (b) kovalent mit einem Metallchelat gekoppelt.

Neben den 20 natürlichen Aminosäuren kann das Peptid auch artefizielle Aminosäuren, wie etwa β-Alanin, γ-Aminobuttersäure, ε-Aminocapronsäure, Norleucin oder Ornithin enthalten. Diese artefiziellen Aminosäuren werden analog wie die natürlichen Aminosäuren für die Synthese in geschützter Form eingesetzt.

Gemäß Variante (a) des erfindungsgemäßen Verfahrens erfolgt nach Beendigung der Synthese die Einführung der Metallchelat-Markierung durch Umsetzung des vorzugsweise festphasengebundenen Peptids mit dem jeweils gewünschten aktivierten Metallchelat, das mit freien primären Aminogruppen der N-terminalen Aminosäure des Peptids reagiert. Pro freie primäre Aminogruppe werden vorzugsweise 1,5 bis 4 Äquivalente Aktivester eingesetzt. Anschließend wird das Reaktionsprodukt, sofern erforderlich, von der Festphase und den Schutzgruppen abgespalten, und dann aufgereinigt, vorzugsweise durch HPLC

Enthält das Peptid noch Aminogruppen, die mit einer zweiten Schutzgruppe, wie etwa Phenylacetyl, derivatisiert sind, so werden diese Schutzgruppen im letzten Schritt entfernt. Die Entfernung von Phenylacetylschutzgruppen kann beispielsweise enzymatisch mit immobilisierter oder löslicher Penicillin G-Amidase in wäßriger Lösung mit organischem Solvensanteil bei Raumtemperatur erfolgen.

Enthalten die durch das erfindungsgemäße Verfahren hergestellten Peptide eine intramolekulare Disulfidbrücke, so kann die Peptidsequenz nach Beendigung der Synthese, aber vor Abspaltung der N-termihalen Fmoc-Schutzgruppe der letzten Aminosäure, z.B. mit Jod in Hexafluorisopropanol/Dichlormethan (Kamber und Hiskey in Gross E. und Meienhofer J., The Peptide Academic Press, New York, 1981, Seiten 145 bis 147) an der Festphase oxidiert, und anschließend die N-terminale Fmoc-Schutzgruppe abgespalten werden.

Es wird ein Peptid synthetisiert, das einen immunologisch reaktiven Epitopbereich, d.h. eine Antikörper-bindende Peptidsequenz, und einen Spacerbereich umfaßt. Es wird mindestens eine Metallchelat-Markierung an den Spacerbereich gekoppelt. Peptide, bei denen die Markierung im Spacerbereich angeordnet ist, zeigen oft eine bessere Sensitivität in immunologischen Tests.

Der Spacerbereich, der vorzugsweise eine Länge von 1 bis 10 Aminosäuren aufweist, wirkt stabilisierend und löslichkeitsvermittelnd, da er vorzugweise Ladungen enthält oder/und Wasserstoffbrücken ausbilden kann. Außerdem kann er die Bindung von mehreren, z.B. hochmolekularen Rezeptoren an das Metallchelat-markierte Peptid sterisch erleichtern. Die Aminosäuren des Spacerbereichs werden vorzugsweise ausgewählt aus der Gruppe bestehend aus Glycin, β-Alanin, γ-Aminobuttersäure, ε-Aminocapronsäure, Lysin und Verbindungen der Strukturformel NH₂-[(CH₂)ₙO]ₓ-C₂-CH₂-COOH, worin n 2 oder 3 ist und x 1 bis 10 ist. Weiterhin enthält der Spacerbereich vorzugsweise mindestens teilweise artefizielle Aminosäurederivate. Der Spacerbereich ist vorzugsweise am Aminoterminus oder/und Carboxyterminus des Peptids angeordnet.

Durch das erfindungsgemäße Verfahren werden vorzugsweise Peptide synthetisiert, die einen Epitopbereich aus pathogenen Organismen, z.B. Bakterien, Viren und Protozoen, oder aus Autoimmun-Antigenen enthalten. Vorzugsweise stammt der immunologisch reaktive Epitopbereich aus viralen Antigenen, z.B. den Aminosäuresequenzen von HIVI, HIVII, HIV-Subtyp O oder Hepatitis C-Virus (HCV). "HIVI (Human Immunodeficiency Virus Typ 1), HIV II (Human Immunodeficiency Virus Typ 2)-, HIV-Subtyp O oder Hepatitis C-Virus (HCV)."

Vorzugsweise werden HIVI-, HIVII- bzw. HIV-Subtyp O-Epitope, aus den Regionen gp32, gp4i und gp120 ausgewählt. HCV-Epitope werden vorzugsweise aus der Core/Env-Region oder den Nicht-Strukturprotein-Regionen NS3, NS4 oder NS5 ausgewählt.

Besonders bevorzugt wird der Epitopbereich von HIVI-, HIVII- oder HIV Subtyp O-Aminosäuresequenzen ausgewählt aus der Gruppe der Aminosäuresequenzen:

| | | | |
|---|---|---|---|
| NNTRKSISIG | PGRAFYT | | (I) |
| NTTRSISIGP | GRAFYT | | (II) |
| IDIQEERRMR | IGPGMAWYS | | (III) |
| QARILAVERY | LKDQQLLGIW | GASG | (IV) |
| LGIWGCSGKL | ICTTAVPWNA | SWS | (V) |
| KDQQLLGIWG | SSGKL | | (VI) |
| ALETLLQNQQ | LLSLW | | (VII) |
| LSLWGCKGKL | VCYTS | | (VIII) |
| WGIRQLRARL | LALETLLQN | | (IX) und |
| QAQLNSWGCA | FRQVCHTTVP | WPNDSLT | (X) |

oder Teilsequenzen davon, die eine Länge von mindestens 6 und vorzugsweise mindestens 8 Aminosäuren aufweisen.

Die Aminosäurensequenzen I bis III stammen aus der gp120-Region von HIVI, die Aminosäuresequenzen IV bis IX stammen aus der gp41 Region von HIVI und die Aminosäuresequenz X stammt aus der gp32-Region von HIVII. Die Aminosäuresequenzen I bis X sind weiterhin in den Sequenzprotokollen SEQ ID NO. 1 bis SEQ ID NO. 10 dargestellt. Die Sequenzen V, VIII und X enthalten jeweils 2 Cysteine, die vorzugsweise in Form einer Disulfidbrücke vorliegen. Vorzugsweise enthalten diese Sequenzen einen N- oder/und C-terminalen Spacer, wie oben definiert, der eine Metallchelat-Markierung trägt. Gegebenenfalls können auch innerhalb des Epitopbereichs liegende Lysinreste in markierter Form vorliegen.

Der Epitopbereich von HCV-Aminosäuresequenzen wird vorzugsweise ausgewählt aus der Gruppe der Aminosäuresequenzen:

| | | | |
|---|---|---|---|
| SRRFAQALPV | WARPD | | (XI) |
| PQDVKFPGGG | QIVGGV | | (XII) |
| EEASQHLPYI | EQ | | (XVII) |
| QKALGLLQT | | | (XIV) |
| SRGNHVSPTH | YVPESDAA | | (XV) |
| PQRKNKRNTN | RRPQDVKFPG | | |
| GGQIVGVV | | | (XVI) und |
| AWYELTPAET | TVRLRAYMNT | PGLPV | (XVII) |

oder Teilsequenzen.davon, die eine Länge von mindestens 6 und vorzugsweise mindestens 8 Aminosäuren aufweisen. Die Sequenz XI stammt aus der NS5-Region, die Sequenzen XII und XVI aus der Core Region, die Sequenzen XIII, XIV und XV aus der NS4 Region und die Sequenz XVII aus der NS3 Region von HCV. Die Aminosäuresequenzen XI bis XVII sind in den Sequenzprotokollen SEQ ID NO. 11 bis SEQ ID NO. 17 dargestellt. Peptide mit den oben genannten Epitopen enthalten zusätzlich einen Spacerbereich, der eine Metallchelat-Markierung trägt.

Ein weiterer Gegenstand der vorliegenden Erfindung ist ein Metallchelat-markiertes Peptid, das eine Länge von maximal 50 und vorzugsweise maximal 30 Aminosäuren aufweist und am Aminoterminus oder/und an Aminoseitengrupen mit mindestens einem lumineszierendem Metallchelat, vorzugsweise einen Metallchelat-Aktivesterderivat gekoppelt ist, wobei das Peptid einen immunologisch reaktiven Epitopbereich und einen Spacerbereich umfasst, wobei mindestens eine Metallchelat-Markierung an den Spacerbereich gekoppelt ist und wobei die Liganden in den Metallchelaten ausgewählt werden aus aromatischen heterocyclischen Polydentatliganden. Vorzugsweise ist das lumineszierende Metallchelat ein Rutheniumchelat.

Das erfindungsgemäße Peptid umfaßt vorzugsweise einen immunologisch reaktiven Epitopbereich, der mit Antikörpern, z.B. aus Humanseren reagieren kann, und einen immunologisch nicht reaktiven Spacerbereich, wobei der Spacerbereich mindestens eine Metallchelatmarkierung trägt. Vorzugsweise ist der Spacerbereich am Aminoterminus des Peptids angeordnet, und hat eine Länge von 1 bis 10 Aminosäuren. Der Epitopbereich stammt vorzugsweise aus den Aminosäuresequenzen von HIVI oder HCVII einschließlich Varianten, z.B. Subtypen davon, z.B. HIV Subtyp O, und ist eine der Aminosäuresequenzen I bis XVII oder eine Teilsequenz davon.

Die vorliegende Erfindung betrifft auch die Verwendung der oben genannten Metallchelat-markierten Peptide als Antigene bei einem immunologischen Verfahren zur Bestimmung von spezifischen Antikörpern in einer Probeflüssigkeit, wobei das Peptid an gezielt ausgewählten Positionen markiert ist. Vorzugsweise werden solche Antikörper bestimmt, die auf eine Infektion durch Mikroorganismen, wie etwa Bakterien, Viren oder Protozoen, hinweisen. Besonders bevorzugt werden zugegen Viren gerichtete Antikörper, z.B. gegen HIV oder Hepatitis-Viren gerichtete Antikörper bestimmt. Die Probeflüssigkeit ist vorzugsweise Serum, besonders bevorzugt humanes Serum. Weiterhin ist bevorzugt, daß die erfindungsgemäßen Metallchelat-markierten Peptide bei einem immunologischen Verfahren im Brückentestformat eingesetzt werden.

Außerdem betrifft die vorliegende Erfindung ein Verfahren zur immunologischen Bestimmung eines spezifischen Antikörpers in einer Probeflüssigkeit, welches dadurch gekennzeichnet ist, daß man die Probeflüssigkeit mit einem ersten markierten, Antigen, das gegen den zu bestimmenden Antikörper gerichtet ist und ein Metallchelat-markiertes Peptid, wie oben definiert, umfaßt inkubiert und den Antikörper über eine Bindung mit dem Peptid nachweist wobei das Peptid an gezielt ausgewählten Positionen markiert ist. Vorzugsweise verwendet man als erstes Antigen ein mit einem Ruthenium-, Rhenium-, Iridium- oder Osmiumchelat markiertes Peptid.

Das erfindungsgemäße immunologische Bestimmungsverfahren kann an sich nach jedem bekannten Testformat erfolgen, z.B. in einem homogenen Immunoassay mit einer einzigen Reaktionsphase oder in einem heterogenen Immunoassay mit mehr als einer Reaktionsphase. Vorzugsweise wird ein heterogenes Testformat verwendet, bei dem das Vorhandensein des Antikörpers in Anwesenheit einer Festphase nachgewiesen wird. Eine Ausführungsform dieses Testformats ist das sogenannte Doppelantigen-Brückentestkonzept. Hierbei wird die Probeflüssigkeit in Gegenwart einer Festphase mit dem ersten Antigen und einem zweiten Antigen inkubiert, das gegen den zu bestimmenden Antikörper gerichtet ist und (a) an die Festphase gebunden ist, oder (b) in einer an die Festphase bindefähigen Form vorliegt. Der zu bestimmende Antikörper in der Probeflüssigkeit wird durch Bestimmung der Markierung in der Festphase oder/und in der flüssigen Phase nachgewiesen. Vorzugsweise ist das zweite Antigen mit Biotin markiert und ist an eine Festphase bindefähig, die mit Streptavidin oder Avidin beschichtet ist. Vorzugsweise verwendet man als zweites Antigen ein mit Biotin markiertes Peptid.

Die Testdurchführung beinhaltet vorzugsweise ein Mischen der Probeflüssigkeit mit dem ersten Antigen und dem festphasenseitigen zweiten Antigen, um einen markierten, immobilisierten Komplex aus erstem Antigen, Antikörper und festphasengebundenem zweiten Antigen zu erhalten. Gegenüber anderen Testformaten zum Nachweis von Antikörpern führt das Brückentestformat sowohl zu einer Verbesserung der Sensitivität, d.h. es werden alle Immunglobulinklassen, wie etwa IgG, IgM, IgA und IgE, erkannt, als auch der Spezifität, d.h. es wird die unspezifische Reaktivität verringert.

Ein weiterer Vorteil des Doppelantigen-Brückentestformats, bei dem ein festphasenseitiges und ein Metallchelat-markiertes Peptid als Antigene eingesetzt werden, besteht in der Möglichkeit der Verringerung des Risikos einer falsch negativen Bewertung von Proben, die einen hohen Titer des zu bestimmenden Antikörpers aufweisen, infolge des Hook-Effekts und zwar durch eine Erhöhung der Anzahl von Markierungsgruppen pro Peptid vorzugsweise auf 2 bis 10 Markierungsgruppen. Die Erhöhung der Anzahl von Metallchelat-Markierungsgruppen führt infolge der Amplifikation des Signals über den Rezeptor zur Verbesserung der Hook-Sensitivität gegenüber Testführungen mit direkt nachweisbaren Markierungsgruppen.

Der Nachweis der lumineszierenden Metallchelatgruppe erfolgt vorzugsweise durch Elektrochemilumineszenz, wobei lumineszierende Spezies elektrochemisch an der Oberfläche einer Elektrode erzeugt werden. Der Nachweis der Lumineszenz kann qualitativ oder/und quantitativ erfolgen. Beispiele zur Durchführung von Lumineszenz-Assays finden sich in EP-A-0 580 979, WO 90/05301, WO 90/11511 und WO 92/14138. Die Festphase in Elektrochemilumineszenz-Assays besteht vorzugsweise aus Mikropartikeln, besonders bevorzugt magnetischen Mikropartikeln, die mit einer Beschichtung versehen sind, die mit dem festphasenseitigen zweiten Antigen wechselwirkt. Vorzugsweise sind die Mikropartikel mit Streptavidin beschichtet.

Die Elektrochemilumineszenz-Messung wird vorzugsweise in Gegenwart eines Reduktionsmittels für den Metallkomplex durchgeführt, z.B. einem Amin. Bevorzugt sind aliphatische Amine, insbesondere primäre, sekundäre und tertiäre Alkylamine, deren Alkylgruppen jeweils ein bis drei Kohlenstoffatome aufweist. Besonders bevorzugt ist Tripropylamin. Das Amin kann jedoch auch ein aromatisches Amin, wie Anilin oder ein heterocyclisches Amin sein.

Weiterhin kann gegebenenfalls als Verstärker ein nichtionisches oberflächenaktives Mittel, z.B. ein ethoxyliertes Phenol vorhanden sein. Derartige Substanzen sind beispielsweise kommerziell unter den Bezeichnungen Triton X100 oder Triton N-401 erhältlich.

Andererseits kann der Nachweis der lumineszierenden Metallchelatgruppe auch durch Fluoreszenz erfolgen, wobei das Metallchelat durch Bestrahlung mit einem Licht der geeigneten Wellenlänge angeregt und die daraus resultierende Fluoreszenzstrahlung gemessen wird. Beispiele zur Durchführung von Fluoreszenz-Assays finden sich in EP-A-0 178 450 und EP-A-0 255 534.

Noch ein weiterer Gegenstand der vorliegenden Erfindung ist ein Reagenz zur immunologischen Bestimmung eines spezifischen Antikörpers, das mindestens ein erfindungsgemäßes Metallchelat-markiertes, mit dem zu bestimmenden Antikörper reagierendes Peptid enthält. Wird das Reagenz in einem Doppelantigen-Brückentest verwendet, so enthält es vorzugsweise (a) das Metallchelat-markierte Peptid, und (b) ein weiteres, mit dem zu bestimmenden Antikörper reagierendes Antigen, das an eine Festphase gebunden ist oder in einer an eine Festphase bindefähigen Form vorliegt.
Schließlich wird auch ein Aminosäurederivat der allgemeinen Formeln (Ia) oder (Ib) offenbart. 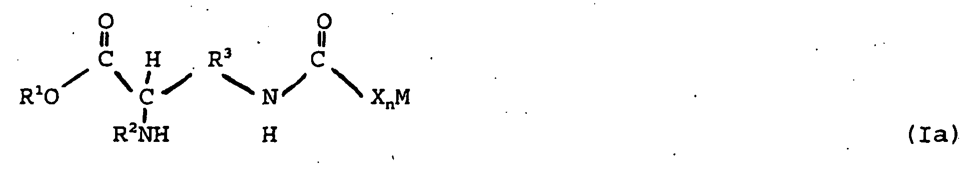 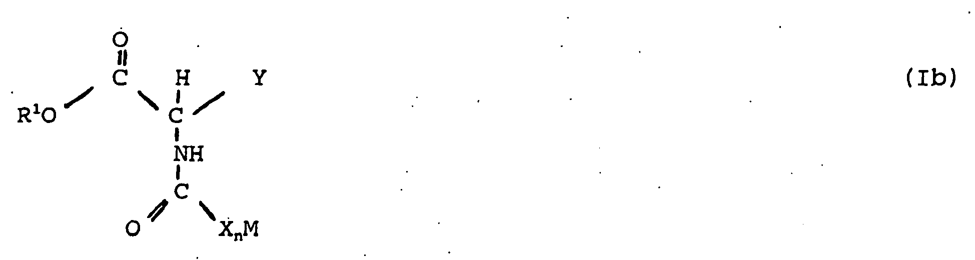 worin:
- R¹: Wasserstoff oder eine kationische Gruppe, z.B. ein Alkalimetall oder ein Ammoniumion, ist,
- R²: Wasserstoff oder eine Aminoschutzgruppe für die Festphasen-Peptidsynthese ist,
- R³: eine C₁-C₅-Alkylengruppe ist,
- Y: die Seitenkette einer beliebigen Aminosäure ist, und
- MXₙ: eine lumineszierende Metallchelatgruppe ist, in der das Metall M durch n gleiche oder verschiedene Liganden X chelatiert ist.

Bei dem Aminosäurederivat der allgemeinen Formel (Ia) ist die Metallchelatgruppe MXₙ an die primäre Aminoseitengruppe einer Aminosäure wie etwa Lysin oder Ornithin gekoppelt. Im Aminosäurederivat der allgemeinen Formel (Ib) ist die Metallchelatgruppe an die α-Aminogruppe einer beliebigen Aminosäure, z.B. einer natürlichen Aminosäure oder einer artefiziellen Aminosäure, die gegebenenfalls eine Schutzgruppe tragen kann, gekoppelt. Aminosäurederivate der allgemeinen Formel (Ia) können innerhalb oder an den Enden der Peptidsequenz eingeführt werden. Aminosäurederivate der allgemeinen Formel (Ib) können, sofern sie keine primäre Aminosäuregruppe im Rest Y enthalten, nur an den N-Terminus der Peptidsequenz eingeführt werden.

Die Metallchelatgruppe MXₙ hat vorzugsweise die Struktur ML₁L₂L₃, wobei L₁, L₂, L₃ gleich oder verschieden sind und jeweils einen Liganden mit mindestens 2 N-haltigen Heterocyclen, z.B. Bipyridyl oder Phenanthrolyl, bedeuten und einer dieser Liganden gegebenenfalls über eine Spacergruppe an eine Aminogruppe der Aminosäure gekoppelt ist..

Ein Beispiel für ein Lysin-Rutheniumchelat (Fmoc-Lys(BPRu)-OH) ist in Fig. 1 gezeigt.

Weiterhin wird die vorliegende Erfindung durch die nachfolgenden Beispiele, Sequenzprotokolle und Figuren beschrieben.

Es zeigen
- SEQ ID NO. 1:: die Aminosäuresequenz eines Epitops aus dem gp120-Bereich von HIVI,
- SEQ ID NO. 2:: die Aminosäuresequenz eines weiteren Epitops aus dem gp120-Bereich von HIVI,
- SEQ ID NO. 3:: die Aminosäuresequenz eines weiteren Epitops aus dem gp120-Bereich von HIVI, Subtyp O;
- SEQ ID NO. 4:: die Aminosäuresequenz des Epitops aus dem gp41-Bereich von HIVI,
- SEQ ID NO. 5:: die Aminosäuresequenz eines weiteren Epitops aus dem gp41-Bereich von HIVI,
- SEQ ID NO. 6:: die Aminosäuresequenz noch eines weiteren Epitops aus dem gp41-Bereich von HIVI,
- SEQ ID NO. 7:: die Aminosäuresequenz eines Epitops aus dem gp41-Bereich von HIVI, Subtyp O;
- SEQ ID NO. 8:: die Aminosäuresequenz eines weiteren Epitops aus dem gp41-Bereich von HIVI, Subtyp O;
- SEQ ID NO. 9:: die Aminosäuresequenz noch eines weiteren Epitops aus dem gp41-Bereich von HIVI, Subtyp O;
- SEQ ID NO.10:: die Aminosäuresequenz eines Epitops aus dem gp32-Bereich von HIVII,
- SEQ ID NO.11:: die Aminosäuresequenz eines Epitops aus dem NS5-Bereich von HCV;
- SEQ ID NO.12:: die Aminosäuresequenz eines Epitops aus dem Core-Bereich von HCV;
- SEQ ID NO.13:: die Aminosäuresequenz eines Epitops aus dem NS4-Bereich von HCV;
- SEQ ID NO.14:: die Aminosäuresequenz eines weiteren Epitops aus dem NS4-Bereich von HCV;
- SEQ ID NO.15:: die Aminosäuresequenz noch eines weiteren Epitops aus dem NS4-Bereich von HCV;
- SEQ ID NO.16:: die Aminosäuresequenz eines weiteren Epitops aus dem Core-Bereich von HCV; und
- SEQ ID NO.17:: die Aminosäuresequenz eines Epitops aus dem NS3-Bereich von HCV und
- Figur 1:: ein Ruthenium(bipyridyl)₃-gekoppeltes und an der α-Aminogruppe geschütztes Lysinderivat.

### Beispiel 1

### Herstellung von Metallchelat-markierten Peptiden

Die Metallchelat-markierten Peptide wurden mittels Fluorenylmethyloxycarbonyl-(Fmoc)-Festphasenpeptidsynthese an einem Batch-Peptidsynthesizer, z.B. von Applied Biosystems A431 oder A433, hergestellt. Dazu wurden jeweils 4.0 Äquivalente der in Tabelle 1 dargestellten Aminosäurederivate verwendet:

**Tabelle 1:**

| | |
|---|---|
| **A** | Fmoc-Ala-OH |
| C | Fmoc-Cys(Trt)-OH |
| D | Fmoc-Asp(OtBu)-OH |
| E | Fmoc-Glu(OtBu)-OH |
| F | Fmoc-Phe-OH |
| G | Fmoc-Gly-OH |
| H | Fmoc-His(Trt)-OH |
| I | Fmoc-Ile-OH |
| K1 | Fmoc-Lys(Boc)-OH |
| K2 | Boc-Lys(Fmoc)-OH |
| K3 | Fmoc-Lys(BPRu)-OH |
| L | Fmoc-Leu-OH |
| M | Fmoc-Met-OH |
| N | Fmoc-Asn(Trt)-OH |
| P | Fmoc-Pro-OH |
| Q | Fmoc-Gln(Trt)-OH |
| R | Fmoc-Arg(Pmc)-OH |
| S | Fmoc-Ser(tBu)-OH |
| T | Fmoc-Thr(tBu)-OH |
| U | Fmoc-βAlanin-OH |
| V | Fmoc-Val-OH |
| W | Fmoc-Trp-OH |
| Y | Fmoc-Tyr(tBu)-OH |
| Z | Fmoc-ε-Aminocapronsäure-OH |
| Nle | Fmoc-ε-Norleucin-OH |
| Abu | Fmoc-γ-Aminobuttersäure-OH |

Bei der Variante (a) - Einführung der Markierung nach Beendigung der Festphasensynthese - wurde aktiviertes BPRu-COOH an die N-terminale Aminosäure des Peptids gekoppelt. Das Lysin-Derivat K1 wurde für den Spacerbereich und das Lysin-Derivat K2 für den Epitopbereich verwendet.

Gemäß Variante (b) erfolgte die Einführung von Metallchelatgruppen in die Peptidsequenz durch direkten Einbau von Metallchelat-gekoppelten Aminosäurederivaten, z.B. innerhalb der Sequenz über einen mit Metallchelat-Aktivester ε-derivatisierten Lysinrest, z.B. das Lysin-Derivat K3 (Fig. 1) oder N-terminal durch Verwendung eines α-derivatisierten Aminosäurerests.

Die Aminosäuren oder Aminosäurederivate wurden in N-Methylpyrrolidon gelöst. Das Peptid wurde an 400-500 mg 4-(2',4'-Dimethoxyphenyl-Fmoc-Aminomethyl)-Phenoxy-Harz (Tetrahedron Letters 28 (1987), 2107) mit einer Beladung von 0,4-0,7 mmol/g aufgebaut (JACS 95 (1973), 1328). Die Kupplungsreaktionen wurden bezüglich des Fmoc-Aminosäurederivats mit 4 Äquivalenten Dicyclohexylcarbodiimid und 4 Äquivalenten N-Hydroxybenzotriazol in Dimethylformamid als Reaktionsmedium während 20 min durchgeführt. Nach jedem Syntheseschritt wurde die Fmoc-Gruppe mit 20%igem Piperidin in Dimethylformamid in 20 min abgespalten.

Bei Anwesenheit von Cysteinresten in der Peptidsequenz erfolgte unmittelbar nach Beendigung der Synthese eine Oxidation an der Festphase mit Jod in Hexafluorisopropanol/Dichlormethan.

Die Freisetzung des Peptids vom Träger und die Abspaltung der säurelabilen Schutzgruppen erfolgte mit 20 ml Trifluoressigsäure, 0,5 ml Ethandithiol, 1 ml Thioanisol, 1,5 g Phenol und 1 ml Wasser in 40 min bei Raumtemperatur. Die Reaktionslösung wurde anschließend mit 300 ml gekühltem Diisopropylether versetzt und zur vollständigen Fällung des Peptids 40 min bei **0°C** gehalten. Der Niederschlag wurde abfiltriert, mit Diisopropylether nachgewaschen, mit wenig 50 %-iger Essigsäure gelöst und lyophilisiert. Das erhaltene Rohmaterial wurde mittels präparativer HPLC an Delta-PAK RP C18-Material (Säule 50 x 300 mm, 100 Å, 15 µ) über einen entsprechenden Gradienten (Eluent A: Wasser, 0,1% Trifluoressigsäure, Eluent B: Acetonitril, 0,1% Trifluoressigsäure) in ca. 120 min. aufgereinigt. Die Identität des eluierten Materials wurde mittels Ionenspray-Massenspektrometrie geprüft.

Die Einführung der Metallchelat-Markierung erfolgte gemäß Variante (a) über entsprechende Aktivester-Derivate an die freie N-terminale Aminogruppe des trägergebundenen Peptids. Hierzu wurden 4 Äquivalente BPRu-COOH pro freie primäre Aminofunktion, aktiviert mit N-Hydroxybenzotriazol/Dicyclohexylcarbodiimid und in wenig DMSO gelöst, zugetropft und bei Raumtemperatur gerührt. Der Umsatz wurde über analytische HPLC verfolgt. Nach Abspaltung vom Träger wurde das Produkt mittels präparativer HPLC aufgereinigt. Die Identität des eluierten Materials wurde mittels Ionenspray-Massenspektrometrie geprüft.

Die Herstellung der Peptide erfolgte auch durch eine Kombination von Variante (a) und (b), d.h. Einbau von Metallchelat-gekoppelten Aminosäurederivaten innerhalb der Sequenz, Abspaltung der N-terminalen Fmoc-Gruppe und Reaktion der freien N-terminalen Aminogruppe mit einem Metallchelat-Aktivesterderivat.

Bei einem ausschließlich direkten Einbau der Metallchelat-gekoppelten Aminosäurederivate während der Festphasensynthese gemäß Variante (b) war eine nachträgliche Einführung von Metallchelat-Aktivestern nicht mehr erforderlich.

Aus den Bereichen gp120, gp41 und gp32 von HIVI bzw. HIVII wurden die in Tabelle 2 dargestellten Peptidverbindungen hergestellt.

**Tabelle 2: Ruthenylierte lineare Peptide**

| | |
|---|---|
| gp120 | BPRu-UZU-NNTRKSISIGPGRAFYT |
| | BPRu-UZ-NTTRSISIGPGRAFY |
| | BPRu(ethylenglykol)-UZ-NTTRSISIGPGRAFY |
| | NNTRKSISIGPGRAFYT-K(BPRu) |
| | BPRu-UZU-IDIQEERRMRIGPGMAWYS |
| gp41/1 | BPRu-UZU-AVERYLKDQQLLGIW |
| | BPRu-UGGG-QARILAVERYLKDQQLLGIWGASG |
| | BPRu-GGGG-QARILAVERYLKDQQLLGIWGASG |
| | BPRu-UZU-WGIRQLRARLLALETLLQN |
| gp41/2 | BPRu-UZU-LGIWGCSGKLICTTAV |
| | BPRu-UGGG-GCSGKLICTTAVPWNASWS |
| | (GCSGKLICTTAVPWNASWS)K-(BPRu) |
| gp41/3 | BPRu-UZU-KDQQLLGIWGSSGKL |
| gp41/4 | BPRu-UZU-ALETLLQNQLLSLW |
| gp32 | BPRu-UZU-NSWGCAFRQVCHTT |
| | BPRu-GGG-QAQLNSWGCAFRQVCHTTVPWPNDSLT |

Aus dem NSS-Bereich, dem NS4-Bereich und dem Core-Bereich von HCV wurden die in der folgenden Tabelle 3 dargestellten Peptide synthetisiert.

**Tabelle 3 : Ruthenylierte lineare Peptide**

| | |
|---|---|
| Core1 | BPRu-GGGG-KNKFNTNRR |
| Core1+2 | BPRu-UZU-KNKRNTNRRPQDVKFPGGGQIVGGV |
| NS4/1+2 | BPRu-UZ-SQHLPYIEQG-NleNle-LAEQFKQQALGLLQT |
| NS4/3m | BPRu-UZ-SRGNHVSPTHYVPESDAA |
| NS5/1 | BPRu-UZ-SRRFAQALPVWARPD |
| Core1+2+3 | BPRu-UZ-KNKRNTNRRPQDVKFPGGGQIVGGVLLPRR |
| Core1m | BPRu-UZ-NPKPQKKNKRNTNRR |
| Core3m | BPRu-UZ-GQIVGGVYLLPRRGPRLG |
| Core2m | BPRu-UZ-PQDVKFPGGGQIVGGV |
| NS4/3m-I | BPRu-UZU-SRGNHVSPTHYVPESDAA |
| NS4/1 | BPRu-UZU-SQHLPYIEQ |

Die Herstellung Biotin-markierter Peptide erfolgte entweder N-terminal durch eine Derivatisierung am Harz (Biotin-Aktivester) oder in die Sequenz über einen mit Biotinaktivester-ε-derivatisierten Lysinrest (Fmoc-Lys (Biotin)-OH).

### Beispiel 2

In einem Doppelantigen-Brückentest wurde ein erfindungsgemäßes Peptidantigen mit einem rekombinanten Polypeptid-Antigen verglichen. In einem erfindungsgemäßen Beispiel wurde das ruthenylierte Peptid-Antigen gp41/2 (Tabelle 2) in Kombination mit einem biotinylierten Peptid-Antigen der gleichen Sequenz getestet. In einem Vergleichsbeispiel wurde ein ruthenyliertes Polypeptid-Antigen rec. gp41 (Chang et al, Science 228 (1985), 93-96) in Kombination mit einem biotinylierten Polypeptid-Antigen der gleichen Sequenz getestet.

Die Ergebnisse dieses Tests zeigten, daß mit dem rekombinanten Polypeptid-Antigen praktisch keine Differenzierung zwischen negativen und positiven Proben möglich ist, während das Peptid-Antigen eine sehr gute Differenzierung erlaubt.

## Patentansprüche

1. Verfahren zur Herstellung von Metallchelat-markierten Peptiden,
**dadurch gekennzeichnet,**
**daß** man
ein Peptid mit der gewünschten Aminosäuresequenz an einer Festphase synthetisiert, das einen immunologisch reaktiven Epitopbereich und einen Spacerbereich umfasst, wobei man
(a) nach der Synthese ein aktiviertes lumineszierendes Metallchelat an die N-terminate primäre Aminogruppe des Peptids koppelt, oder/und
(b) während der Synthese an mindestens einer Position des Peptids ein Aminosäurederivat einführt, das kovalent mit einer lumineszierenden Metallchelat-Markierungsgruppe gekoppelt ist,
wobei mindestens eine Metallchelat-Markierung an den Spacerbereich gekoppelt wird und wobei die Liganden in den Metallchelaten ausgewählt werden aus aromatischen heterocyclischen Polydentatliganden.

2. Verfahren nach Anspruch 1,
**dadurch gekennzeichnet,**
**daß** das lumineszierende Metallchelat ausgewählt wird aus der Gruppe bestehend aus Ruthenium-, Rhenium-, Iridium- und Osmiumchelaten.

3. Verfahren nach einem der Ansprüche 1 bis 2,
**dadurch gekennzeichnet,**
**daß** man ein Peptid synthetisiert, das einen immunologisch reaktiven viralen Epitopbereich enthält.

4. Verfahren nach Anspruch 3,
**dadurch gekennzeichnet,**
**daß** der Epitopbereich ausgewählt wird aus der Gruppe der HIVI-(Human Immunodeficiency Virus Typ 1) und HIVII- (Human Immunodeficiency Virus Typ 2) Aminosäuresequenzen
| | | | |
|---|---|---|---|
| NNTRKSISIG | PGRAFYT | | (I) |
| NTTRSISIGP | GRAFYT | | (II) |
| IDIQEERRMR | IGPGMAWYS | | (III) |
| QARILAVERY | LKDQQLLGIW | GASG | (IV) |
| LGIWGCSGKL | ICTTAVPWNA | SWS | (V) |
| KDQQLLGIWG | SSGKL | | (VI) |
| ALETLLQNQQ | LLSLW | | (VII) |
| LSLWGCKGKL | VCYTS | | (VIII) |
| WGIRQLRARL | LALETLLQN | | (IX) und |
| QAQLNSWGCA | FRQVCHTTVP | WPNDSLT | (X) |
oder Teilsequenzen davon, die eine Länge von mindestens 6 Aminosäuren aufweise.

5. Verfahren nach Anspruch 3,
**dadurch gekennzeichnet,**
**daß** der Epitopbereich ausgewählt wird aus der Gruppe der HCV-(Hepatitis-C-Virus) Aminosäuresequenzen
| | | | |
|---|---|---|---|
| SRRFAQALPV | WARPD | | (XI) |
| PQDVKFPGGG | QIVGGV | | (XII) |
| EEASQHLPYI | EQ | | (XVII) |
| QKALGLLQT | | | (XIV) |
| SRGNHVSPTH | YVPESDAA | | (XV) |
| PQRKNKRNTN | RRPQDVKFPG | | |
| GGQIVGVV | | | (XVI) und |
| AWYELTPAET | TVRLRAYMNT | PGLPV | (XVII) |
oder Teilsequenzen davon, die eine Länge von mindestens 6 Aminosäuren aufweisen.

6. Metallchelat-markiertes Peptid, das eine Länge von maximal 50 Aminosäuren aufweist und am Aminoterminus oder/und an Aminoseitengruppen an gezielt ausgewählten Positionen mit mindestens einem lumineszierenden Metallchelat gekoppelt ist, wobei das Peptid einen immunologisch reaktiven Epitopbereich und einen Spacerbereich umfasst, wobei mindestens eine Metallchelat-Markierung an den Spacerbereich gekoppelt ist und wobei die Liganden in den Metallchelaten ausgewählt werden aus aromatischen heterocyclischen polydentatliganden.

7. Peptid nach Anspruch 6,
**dadurch gekennzeichnet,**
**daß** der Epitopbereich ausgewählt ist aus der Gruppe der HIVI- und HIVII-Aminosäuresequenzen
| | | | |
|---|---|---|---|
| NNTRKSISIG | PGRAFYT | | (I) |
| NTTRSISIGP | GRAFYT | | (II) |
| IDIQEERRMR | IGPGMAWYS | | (III) |
| QARILAVERY | LKDQQLLGIW | GASG | (IV) |
| LGIWGCSGKL | ICTTAVPWNA | SWS | (V) |
| KDQQLLGIWG | SSGKL | | (VI) |
| ALETLLQNQQ | LLSLW | | (VII) |
| LSLWGCKGKL | VCYTS | | (VIII) |
| WGIRQLRARL | LALETLLQN | | (IX) und |
| QAQLNSWGCA | FRQVCHTTVP | WPNDSLT | (X) |
oder Teilsequenzen davon, die eine Länge von mindestens 6 Aminosäuren aufweisen.

8. Peptid nach Anspruch 6,
**dadurch gekennzeichnet,**
**daß** der Epitopbereich ausgewählt ist aus der Gruppe der HCV-Aminosäuresequenzen
| | | | |
|---|---|---|---|
| SRRFAQALPV | WARPD | | (XI) |
| PQDVKFPGGG | QIVGGV | | (XII) |
| EEASQHLPYI | EQ | | (XIII) |
| QKALGLLQT | | | (XIV) |
| SRGNHVSPTH | YVPESDAA | | (XV) |
| PQRKNKRNTN | RRPQDVKFPG | | |
| GGQIVGGV | | | (XVI) und |
| AWYELTPAET | TVRLRAYMNT | PGLPV | (XVII) |
oder Teilsequenzen davon, die eine Länge von mindestens 6 Aminosäuren aufweisen.

9. Verwendung von Metallchelat-markierten Peptiden, die durch ein Verfahren nach einem der Ansprüche 1 bis 5 hergestellt wurden, oder von Peptiden nach einem der Ansprüche 6 bis 8 als Antigene bei einem immunologischen Verfahren zur Bestimmung von spezifischen Antikörpern in einer Probeflüssigkeit, wobei das Peptid an gezielt ausgewählten Positionen markiert ist.

10. Verfahren zur immunologischen Bestimmung eines spezifischen Antikörpers in einer Probeflüssigkeit,
**dadurch gekennzeichnet,**
**dass** man die Pobeflüssigkeit mit einem ersten markierten Antigen, das gegen den zu bestimmenden Antikörper gerichtet ist und ein Metallchelat-markiertes Peptid, das durch, ein Verfahren nach einem der Ansprüche 1 bis 5 hergestellt wurde, oder ein Peptid nach einem der Ansprüche 6 bis 8 umfasst, inkubiert und den Antikörper über eine Bindung mit dem Peptid nachweist, wobei das Peptid an gezielt ausgewählten Positionen markiert ist.

11. Verfahren nach Anspruch 10,
**dadurch gekennzeichnet,**
**dass** man die Probeflüssigkeit in Gegenwart einer Festphase mit dem ersten Antigen und einem zweiten Antigen inkubiert, das gegen den zu bestimmenden Antikörper gerichtet ist und
(a) an die Festphase gebunden ist oder
(b) in einer an die Festphase bindefähigen Form vorliegt, und den zu bestimmenden Antikörper durch Bestimmung der Markierung in der Festphase oder/und in der flüssigen Phase nachweist.

12. Reagenz zur immunologischen Bestimmung eines spezifischen Antikörpers,
**dadurch gekennzeichnet,**
**dass** es mindestens ein Metallchelat-markiertes, mit dem zu bestimmenden Antikörper reagierendes Peptid, das durch ein Verfahren nach einem der Ansprüche 1 bis 5 hergestellt wurde, oder ein Metallchelat-markiertes, mit dem zu bestimmenden Antikörper reagierendes Peptid nach einem der Ansprüche 6 bis 8 enthält, wobei das Peptid an gezielt ausgewählten Positionen markiert ist.

13. Reagenz nach Anspruch 12,
**dadurch gekennzeichnet,**
**dass** es
(a) das Metallchelat-markierte, mit dem zu bestimmenden Antikörper reagierende Peptid, und
(b) ein weiteres, mit dem zu bestimmenden Antikörper reagierendes Antigen, das an eine Festphase gebunden ist oder in einer an eine Festphase bindefähige Form vorliegt, enthält.

## Claims

1. Process for the production of metal chelate-labelled peptides,
**characterized in that**
a peptide having the desired amino acid sequence and comprising an immunologically reactive epitope region and a spacer region is synthesized on a solid phase in which
(a) after the synthesis an activated luminescent metal chelate is coupled to the N-terminal primary amino group of the peptide or/and,
(b) an amino acid derivative which is covalently coupled to a luminescent metal chelate marker group is introduced during the synthesis at at least one position of the peptide,
wherein at least one metal chelate label is coupled to the spacer region and wherein the ligands in the metal chelates are selected from aromatic heterocyclic polydentate ligands.

2. Process according to claim 1,
**characterized in that**
the luminescent metal chelate is selected from the group comprising ruthenium, rhenium, iridium and osmium chelates.

3. Process according to one of the claims 1 to 2,
**characterized in that**
a peptide is synthesized which contains an immunologically reactive viral epitope region.

4. Process according to claim 3,
**characterized in that**
the epitope region is selected from the group of HIV I (human immunodeficiency virus type 1) and HIV II (human immunodeficiency virus type 2) amino acid sequences
| | | | |
|---|---|---|---|
| NNTRKSISIG | PGRAFYT | | (I) |
| NTTRSISIGP | GRAFYT | | (II) |
| IDIQEERRMR | IGPGMAWYS | | (III) |
| QARILAVERY | LKDQQLLGIW | GASG | (IV) |
| LGIWGCSGKL | ICTTAVPWNA | SWS | (V) |
| KDQQLLGIWG | SSGKL | | (VI) |
| ALETLLQNQQ | LLSLW | | (VII) |
| LSLWGCKGKL | VCYTS | | (VIII) |
| WGIRQLRARL | LALETLLQN | | (IX) and |
| QAQLNSWGCA | FRQVCHTTVP | WPNDSLT | (X) |
or partial sequences thereof which have a length of at least 6 amino acids.

5. Process according to claim 3,
**characterized in that**
the epitope region is selected from the group of HCV (hepatitis C virus) amino acid sequences
| | | | |
|---|---|---|---|
| SRRFAQALPV | WARPD | | (XI) |
| PQDVKFPGGG | QIVGGV | | (XII) |
| EEASQHLPYI | EQ | | (XVII) |
| QKALGLLQT | | | (XIV) |
| SRGNHVSPTH | YVPESDAA | | (XV) |
| PQRKNKRNTN | RRPQDVKFPG | | |
| GGQIVGVV | | | (XVI) and |
| AWYELTPAET | TVRLRAYMNT | PGLPV | (XVII) |
or partial sequences thereof which have a length of at least 6 amino acids.

6. Metal chelate-labelled peptide which has a maximum length of 50 amino acids and is coupled to at least one luminescent metal chelate at the amino terminus or/and at amino side groups at specifically selected positions, wherein the peptide comprises an immunologically reactive epitope region and a spacer region, wherein at least one metal chelate marker is coupled to the spacer region and wherein the ligands in the metal chelates are selected from aromatic heterocyclic polydentate ligands.

7. Peptide according to claim 6,
**characterized in that**
the epitope region is selected from the group of HIV I or HIV II amino acid sequences
| | | | |
|---|---|---|---|
| NNTRKSISIG | PGRAFYT | | (I) |
| NTTRSISIGP | GRAFYT | | (II) |
| IDIQEERRMR | IGPGMAWYS | | (III) |
| QARILAVERY | LKDQQLLGIW | GASG | (IV) |
| LGIWGCSGKL | ICTTAVPWNA | SWS | (V) |
| KDQQLLGIWG | SSGKL | | (VI) |
| ALETLLQNQQ | LLSLW | | (VII) |
| LSLWGCKGKL | VCYTS | | (VIII) |
| WGIRQLRARL | LALETLLQN | | (IX) and |
| QAQLNSWGCA | FRQVCHTTVP | WPNDSLT | (X) |
or partial sequences thereof which have a length of at least 6 amino acids.

8. Peptide according to claim 6,
**characterized in that**
the epitope region is selected from the group of HCV amino acid sequences
| | | | |
|---|---|---|---|
| SRRFAQALPV | WARPD | | (XI) |
| PQDVKFPGGG | QIVGGV | | (XII) |
| EEASQHLPYI | EQ | | (XIII) |
| QKALGLLQT | | | (XIV) |
| SRGNHVSPTH | YVPESDAA | | (XV) |
| PQRKNKRNTN | RRPQDVKFPG | | |
| GGQIVGGV | | | (XVI) and |
| AWYELTPAET | TVRLRAYMNT | PGLPV | (XVII) |
or partial sequences thereof which have a length of at least 6 amino acids.

9. Use of metal chelate-labelled peptides which have been produced by a process according to one of the claims 1 to 5 or of peptides according to one of the claims 6 to 8 as antigens in an immunological method for the determination of specific antibodies in a sample liquid, wherein the peptide is labelled at specifically selected positions.

10. Method for the immunological determination of a specific antibody in a sample liquid,
**characterized in that**
the sample liquid is incubated with a first labelled antigen which is directed against the antibody to be determined and comprises a metal chelate-labelled peptide which has been produced by a process according to one of the claims 1 to 5 or a peptide according to one of the claims 6 to 8 and the antibody is detected via binding to the peptide, wherein the peptide is labelled at specifically selected positions.

11. Method according to claim 10,
**characterized in that**
the sample liquid is incubated in the presence of a solid phase with the first antigen and with a second antigen which is directed against the antibody to be determined and
(a) is bound to the solid phase or
(b) is present in a form capable of binding to the solid phase and the antibody to be determined is detected by determining the label in the solid phase or/and in the liquid phase.

12. Reagent for the immunological determination of a specific antibody,
**characterized in that**
it contains at least one metal chelate-labelled peptide that reacts with the antibody to be determined which has been produced by a process according to one of the claims 1 to 5 or contains a metal chelate-labelled peptide which reacts with the antibody to be determined according to one of the claims 6 to 8, wherein the peptide is labelled at specifically selected positions.

13. Reagent according to claim 12,
**characterized in that**
it contains
(a) the metal chelate-labelled peptide that reacts with the antibody to be determined and,
(b) a further antigen that reacts with the antibody to be determined which is bound to a solid phase or is present in a form which is capable of binding to a solid phase.

## Revendications

1. Procédé de production de peptides marqués par des chélates métalliques,
**caractérisé en ce**
**que** l'on synthétise
un peptide présentant la séquence d'acides aminés souhaitée sur une phase solide qui comprend une région d'épitope immunologiquement réactive et une région d'espacement, en
(a) couplant après la synthèse, un chélate métallique luminescent activé au groupe amino primaire N-terminal du peptide et/ou
(b) introduisant un dérivé d'acide aminé pendant la synthèse au niveau d'une position du peptide, qui est couplé de façon covalente avec un groupe de marquage par chélates métalliques luminescents,
au moins un marquage par chélates métalliques étant couplé à la région d'espacement, les ligands dans les chélates métalliques étant choisis parmi les ligands polydentates hétérocycliques aromatiques.

2. Procédé selon la revendication 1,
**caractérisé en ce**
**que** le chélate métallique luminescent est choisi dans le groupe comprenant les chélates de ruthénium, rhénium, iridium et osmium.

3. Procédé selon l'une des revendications 1 à 2,
**caractérisé en ce**
**que** l'on synthétise un peptide qui contient une région d'épitope viral immunologiquement réactive.

4. Procédé selon la revendication 3,
**caractérisé en ce**
**que** la région d'épitope est choisie dans le groupe des séquences d'acides aminés de VIH I (virus de l'immunodéficience humaine de type 1) et VIH II (virus de l'immunodéficience humaine de type 2)
| | | | |
|---|---|---|---|
| NNTRKSISIG | PGRAFYT | | (I) |
| NTTRSISIGP | GRAFYT | | (II) |
| IDIQEERRMR | IGPGMAWYS | | (III) |
| QARILAVERY | LKDQQLLGIW | GASG | (IV) |
| LGIWGCSGKL | ICTTAVPWNA | SWS | (V) |
| KDQQLLGIWG | SSGKL | | (VI) |
| ALETLLQNQQ | LLSLW | | (VII) |
| LSLWGCKGKL | VCYTS | | (VIII) |
| WGIRQLRARL | LALETLLQN | | (IX) et |
| QAQLNSWGCA | FRQVCHTTVP | WPNDSLT | (X) |
ou des séquences partielles de ceux-ci, qui présentent une longueur d'au moins 6 acides aminés.

5. Procédé selon la revendication 3,
**caractérisé en ce**
**que** la région d'épitope est choisie dans le groupe des séquences d'acides aminés de HCV (virus de l'hépatite C)
| | | | |
|---|---|---|---|
| SRRFAQALPV | WARPD | | (XI) |
| PQDVKFPGGG | QIVGGV | | (XII) |
| EEASQHLPYI | EQ | | (XVII) |
| QKALGLLQT | | | (XIV) |
| SRGNHVSPTH | YVPESDAA | | (XV) |
| PQRKNKRNTN | RRPQDVKFPG | | |
| GGQIVGVV | | | (XVI) et |
| AWYELTPAET | TVRLRAYMNT | PGLPV | (XVII) |
ou des séquences partielles de ceux-ci qui présentent une longueur d'au moins 6 acides aminés.

6. Peptide marqué par un chélate métallique qui présente une longueur maximale de 50 acides aminés et est couplé à une terminaison amino et/ou un groupe latéral amino sur des positions choisies de façon ciblée avec au moins un chélate métallique luminescent, le peptide comprenant une région d'épitope immunologiquement réactive et une région d'espacement et au moins un marquage par chélates métalliques étant couplé au niveau de la région d'espacement, les ligands dans les chélates métalliques étant choisis parmi les ligands polydentates hétérocycliques aromatiques.

7. Peptide selon la revendication 6,
**caractérisé en ce**
**que** la région d'épitope est choisie dans le groupe des séquences d'acides aminés de VIH I et VIH II
| | | | |
|---|---|---|---|
| NNTRKSISIG | PGRAFYT | | (I) |
| NTTRSISIGP | GRAFYT | | (II) |
| IDIQEERRMR | IGPGMAWYS | | (III) |
| QARILAVERY | LKDQQLLGIW | GASG | (IV) |
| LGIWGCSGKL | ICTTAVPWNA | SWS | (V) |
| KDQQLLGIWG | SSGKL | | (VI) |
| ALETLLQNQQ | LLSLW | | (VII) |
| LSLWGCKGKL | VCYTS | | (VIII) |
| WGIRQLRARL | LALETLLQN | | (IX) et |
| QAQLNSWGCA | FRQVCHTTVP | WPNDSLT | (X) |
ou les séquences partielles de ceux-ci qui présentent une longueur d'au moins 6 acides aminés.

8. Peptide selon la revendication 6,
**caractérisé en ce**
**que** la région d'épitope est choisie dans le groupe des séquences d'acides aminés de HCV
| | | | |
|---|---|---|---|
| SRRFAQALPV | WARPD | | (XI) |
| PQDVKFPGGG | QIVGGV | | (XII) |
| EEASQHLPYI | EQ | | (XIII) |
| QKALGLLQT | | | (XIV) |
| SRGNHVSPTH | YVPESDAA | | (XV) |
| PQRKNKRNTN | RRPQDVKFPG | | |
| GGQIVGGV | | | (XVI) et |
| AWYELTPAET | TVRLRAYMNT | PGLPV | (XVII) |
ou des séquences partielles de celles-ci qui présentent une longueur d'au moins 6 acides aminés.

9. Utilisation de peptides marqués par un chélate métallique qui a été produit par un procédé selon l'une des revendications 1 à 5 ou de peptides selon l'une des revendications 6 à 8 comme antigènes dans un procédé immunologique de détermination d'anticorps spécifiques dans un liquide d'échantillon, le peptide étant marqué au niveau de positions ciblées choisies.

10. Procédé de détermination immunologique d'un anticorps spécifique dans un liquide d'échantillon,
**caractérisé en ce**
**que** l'on incube le liquide d'échantillon avec un premier antigène marqué qui est dirigé contre l'anticorps à déterminer et un peptide marqué par un chélate qui a été produit par un procédé selon l'une des revendications 1 à 5 ou comprend un peptide selon l'une des revendications 6 à 8 et on détecte l'anticorps par une liaison avec le peptide, le peptide étant marqué sur des positions choisies de façon ciblée.

11. Procédé selon la revendication 10,
**caractérisé en ce**
**que** l'on incube le liquide d'échantillon en présence d'une phase solide avec le premier antigène et un deuxième antigène qui est dirigé contre l'anticorps à déterminer et
(a) est lié à la phase solide ou
(b) se présente sous une forme pouvant se lier à la phase solide et détecte les anticorps à déterminer par détermination du marquage dans la phase solide et/ou dans la phase liquide.

12. Réactif de détermination immunologique d'un anticorps spécifique,
**caractérisé en ce**
**qu'**il contient au moins un peptide réagissant avec l'anticorps à déterminer, marqué par le chélate métallique qui a été produit par un procédé selon l'une des revendications 1 à 5 ou un peptide réagissant avec l'anticorps à déterminer, marqué par le chélate métallique selon l'une des revendications 6 à 8, le peptide étant marqué au niveau de positions choisies de façon ciblée.

13. Réactif selon la revendication 12,
**caractérisé en ce**
(a) **qu'**il contient le peptide réagissant avec l'anticorps à déterminer, marqué par le chélate métallique, et
(b) **qu'**un autre antigène réagissant avec l'anticorps à déterminer qui est lié à une phase solide ou se présente sous une forme pouvant se lier à une phase solide.


### SEQUENZPROTOKOLL

(1) ALLGEMEINE INFORMATION:
   (i) ANMELDER:
      (A) NAME: Boehringer Mannheim GmbH
      (B) STRASSE: Sandhofer Str. 116
      (C) ORT: Mannheim
      (E) LAND: Deutschland
      (F) POSTLEITZAHL: 68305
   (ii) ANMELDETITEL: Metallchelat-markiertePeptide
   (iii) ANZAHL DER SEQUENZEN: 17
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.25 (EPA)
(2) INFORMATION ZU SEQ ID NO: 1:
   (i) SEQUENZ CHARAKTERISTIKA:
      (A) LÄNGE: 17 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
   (viii) POSITION IM GENOM:
      (A) CHROMOSOM/SEGMENT:gp120
   (xi) SEQUENZBESCHREIBUNG:SEQ ID NO: 1:
   Asn Asn Thr Arg Lys Ser Ile Ser Ile Gly Pro Gly Arg Ala Phe Tyr
   1               5                   10                  15
   Thr
(2) INFORMATION ZU SEQ ID NO: 2:
   (i) SEQUENZ CHARAKTERISTIKA:
      (A) LÄNGE: 16 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
   (viii) POSITION IM GENOM:
      (A) CHROMOSOM/SEGMENT:gp120
   (xi) SEQUENZBESCHREIBUNG:SEQ ID NO: 2:
   Asn Thr Thr Arg Ser Ile Ser Ile Gly Pro Gly Arg Ala Phe Tyr Thr
   1 5 10 15
(2) INFORMATION ZU SEQ ID NO: 3:
   (i) SEQUENZ CHARAKTERISTIKA:
      (A) LÄNGE: 19 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
      (B) STAMM: Subtype O
   (viii) POSITION IM GENOM:
      (A) CHROMOSOM/SEGMENT:gp120
   (xi) SEQUENZBESCHREIHUNG:SEQ ID NO: 3:
   Ile Asp Ile Gln Glu Glu Arg Arg Met Arg Ile Gly Pro Gly Met Ala
   1 5 10 15
   Trp Tyr Ser
(2) INFORMATION ZU SEQ ID NO: 4:
   (i) SEQUENZ CHARAKTERISTIKA:
      (A) LÄNGE: 24 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiencyvirus type 1
   (viii) POSITION IM GENOM:
      (A) CHROMOSOM/SEGMENT: gp41
   (xi) SEQUENZBESCHREIBUNG:SEQ ID NO: 4:
   Gln Ala Arg Ile Leu Ala Val Glu Arg Tyr Leu Lys Asp Gln Gln Leu
   1 5 10 15
   Leu Gly Ile Trp Gly Ala Ser Gly
   20
(2) INFORMATION ZU SEQ ID NO: 5:
   (i) SEQUENZ CHARAKTERISTIKA:
      (A) LÄNGE: 23 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
   (viii) POSITION IM GENOM:
      (A) CHROMOSOM/SEGMENT: gp41
   (xi) SEQUENZHESCHREIBUNG:SEQ ID NO: 5:
(2) INFORMATION ZU SEQ ID NO: 6:
   (i) SEQUENZ CHARAKTERISTIKA:
      (A) LÄNGE: 15 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiencyvirus type 1
   (viii) POSITION IM GENOM:
      (A) CHROMOSOM/SEGMENT: gp41
   (xi) SEQUENZBESCHREIHUNG:SEQ ID NO: 6:
   Lys Asp Gln Gln Leu Leu Gly Ile Trp Gly Ser Ser Gly Lys Leu
   1 5 10 15
(2) INFORMATION ZU SEQ ID NO: 7:
   (i) SEQUENZ CHARAKTERISTIKA:
      (A) LÄNGE: 15 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiencyvirus type 1
      (B) STAMM: Subtype-O
   (viii) POSITION IM GENOM:
      (A) CHROMOSOM/SEGMENT: gp41
   (xi) SEQUENZBESCHREIBUNG:SEQ ID NO: 7:
   Ala Leu Glu Thr Leu Leu Gln Asn Gln Gln Leu Leu Ser Leu Trp
   1 5 10 15
(2) INFORMATION ZU SEQ ID NO: 8:
   (i) SEQUENZ CHARAKTERISTIKA:
      (A) LÄNGE: 15 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiencyvirus type 1
      (B) STAMM: Subtype O
   (viii) POSITION IM GENOM:
      (A) CHROMOSOM/SEGMENT: gp41
   (xi) SEQUENZBESCHREIHUNG:SEQ ID NO: 8:
   Leu Ser Leu Trp Gly Cys Lys Gly Lys Leu Val Cys Tyr Thr Ser
   1 5 10 15
(2) INFORMATION ZU SEQ ID NO: 9:
   (i) SEQUENZ CHARAKTERISTIKA:
      (A) LÄNGE: 19 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiency virus type 1
      (B) STAMM: Subtype O
   (viii) POSITION IM GENOM:
      (A) CHROMOSOM/SEGMENT: gp41
   (xi) SEQUENZBESCHREIBUNG: SEQ ID NO: 9:
   Trp Gly Ile Arg Gln Leu Arg Ala Arg Leu Leu Ala Leu Glu Thr Leu
   1 5 10 15
   Leu Gln Asn
(2) INFORMATION ZU SEQ ID NO: 10:
   (i) SEQUENZ CHARAKTERISTIKA:
      (A) LÄNGE: 27 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Human immunodeficiencyvirus type 2
   (viii) POSITION IM GENOM:
      (A) CHROMOSOM/SEGMENT: gp32
   (xi) SEQUENZBESCHREIBUNG: SEQ ID NO: 10:
   Gln Ala Gln Leu Asn Ser Trp Gly Cys Ala Phe Arg Gln Val Cys His
   1 5 10 15
   Thr Thr Val Pro Trp Pro Asn Asp Ser Leu Thr
   20 25
(2) INFORMATION ZU SEQ ID NO: 11:
   (i) SEQUENZ CHARAKTERISTIKA:
      (A) LÄNGE: 15 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C virus
   (viii) POSITION IM GENOM:
      (A) CHROMOSOM/SEGMENT: NS5
   (xi) SEQUENZBESCHREIBUNG:SEQ ID NO: 11:
   Ser Arg Arg Phe Ala Gln Ala Leu Pro Val Trp Ala Arg Pro Asp
   1 5 10 15
(2) INFORMATION ZU SEQ ID NO: 12:
   (i) SEQUENZ CHARAKTERISTIKA
      (A) LÄNGE: 16 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C virus
   (viii) POSITION IM GENOM:
      (A) CHROMOSOM/SEGMENT: Core
   (xi) SEQUENZBESCHREIBUNG:SEQ ID NO: 12:
   Pro Gln Asp Val Lys Phe Pro Gly Gly Gly Gln Ile Val Gly Gly Val
   1 5 10 15
(2) INFORMATION ZU SEQ ID NO: 13:
   (i) SEQUENZ CHARAKTERISTIKA:
      (A) LÄNGE: 12 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C virus
   (viii) POSITION IM GENOM:
      (A) CHROMOSOM/SEGMENT: NS4
   (xi) SEQUENZBESCHREIBUNG: SEQ ID NO: 13:
   Glu Glu Ala Ser Gln His Leu Pro Tyr Ile Glu Gln
   1 5 10
(2) INFORMATION ZU SEQ ID NO: 14:
   (i) SEQUENZ CHARAKTERISTIKA:
      (A) LÄNGE: 9 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C Virus
   (viii) POSITION IM GENOM:
      (A) CHROMOSOM/SEGMENT: NS4
   (xi) SEQUENZBESCHREIBUNG: SEQ ID NO: 14:
   Gln Lys Ala Leu Gly Leu Leu Gln Thr
   1 5
(2) INFORMATION ZU SEQ ID NO: 15:
   (i) SEQUENZ CHARAKTERISTIKA:
      (A) LÄNGE: 18 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C Virus
   (viii) POSITION IM GENOM:
      (A) CHROMOSOM/SEGMENT: NS4
   (xi) SEQUENZBESCHREIBUNG: SEQ ID NO: 15:
   Ser Arg Gly Asn His Val Ser Pro Thr His Tyr Val Pro Glu Ser Asp
   1               5                   10                  15
   Ala Ala
(2) INFORMATION ZU SEQ ID NO: 16:
   (i) SEQUENZ CHARAKTERISTIKA:
      (A) LÄNGE: 28 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS : Hepatitis C Virus
   (viii) POSITION IM GENOM:
      (A) CHROMOSOM/SEGMENT:Core
   (xi) SEQUENZBESCHREIBUNG: SEQ ID NO: 16:
   Pro Gln Arg Lys Asn Lys Arg Asn Thr Asn Arg Arg Pro Gln Asp Val
   1               5                   10                  15
   Lys Phe Pro Gly Gly Gly Gln Ile Val Gly Val Val
               20                  25
(2) INFORMATION ZU SEQ ID NO: 17:
   (i) SEQUENZ CHARAKTERISTIKA:
      (A) LÄNGE: 25 Aminosäuren
      (B) ART: Aminosäure
      (D) TOPOLOGIE: linear
   (ii) ART DES MOLEKÜLS: Peptid
   (iii) HYPOTHETISCH: NEIN
   (vi) URSPRÜNGLICHE HERKUNFT:
      (A) ORGANISMUS: Hepatitis C Virus
   (viii) POSITION IM GENOM:
      (A) CHROMOSOM/SEGMENT: NS3
   (xi) SEQUENZBESCHREIBUNG: SEQ ID NO: 17:
   Ala Trp Tyr Glu Leu Thr Pro Ala Glu Thr Thr Val Arg Leu Arg Ala
   1               5                   10                  15
   Tyr Met Asn Thr Pro Gly Leu Pro Val
               20                  25